# EUROPEAN PATENT SPECIFICATION

(11) **EP 3 145 423 B1**
(45) Date of publication and mention of the grant of the patent: **30.12.2020**
(21) Application number: 15729609.6
(22) Date of filing: 19.05.2015
(51) Int. Cl.: A61B 17/12, A61F 2/90, A61F 2/97

(54) **ANEURYSM STOP PRESSURE SYSTEM**
DRUCKSYSTEM FÜR ANEURYSMENSTOPP
SYSTÈME DE PRESSION POUR BLOCAGE D'UN ANÉVRISME

(30) Priority: 20.05.2014 US 201462000819 P
(43) Date of publication of application: 29.03.2017
(73) Proprietor: Muffin Incorporated, West Lafayette, IN 47906 (US)
(72) Inventor: FEARNOT, Neal, E., West Lafayette, IN 47906 (US); HANSEN, Palle, Munk, 4632 Bjaeverskov (DK)
(74) Representative: Mathys & Squire
(86) International application number: PCT/US2015/031546
(87) International publication number: WO 2015/179377

(56) References cited:
- WO-A2-2009/019664
- US-A1- 2002 169 473
- US-A1- 2006 287 712
- US-A1- 2007 191 884
- US-A1- 2007 233 220
- US-A1- 2008 114 436
- US-A1- 2010 063 531
- US-A1- 2010 160 949
- US-A1- 2011 160 833

## Description

### FIELD OF THE INVENTION

The present disclosure generally pertains to the field of implantable medical devices. More particularly, the present invention relates to closure devices and methods for blocking blood flow into an aneurysm.

### BACKGROUND

Aneurysms are dilations that are caused from weakening of a blood vessel wall. The dilation is produced by the pressure exerted by blood flow, which causes the weakened segment of the blood vessel, such as an artery or vein, to expand. Aneurysms, including cerebral aneurysms, oftentimes occur in areas where there is a change in direction of blood flow, such as at a bifurcation of the vessel or at a bend in the vessel. The inertia of the moving blood can cause portions of the vessel wall to experience higher shear stress and can increase turbulent flow in the vessel. It is in these areas that aneurysms are more likely to form. In patients experiencing high blood pressure, the stress on the vessel wall is even greater as is the risk of an aneurysm being formed and rupturing.

In some types of aneurysms, such as intracranial aneurysms, this expansion may result in a balloon-like polyp (sometimes referred to as berry aneurysm). In other cases the expansion causes a circumscribed bulge in the blood vessel, as is the case with aortic aneurysms. Continued growth and/or eventual rupture of the ballooned arterial wall can have devastating results for patients. Consequently, unruptured aneurysms are usually treated to prevent hemorrhage, while ruptured aneurysms are typically treated to avert re-rupture and additional concomitant damage.

One surgical intervention for weakened, aneurysmal, or ruptured vessels involves the use of an endoluminal prosthesis such as a stent graft. Such a prosthesis may provide some or all of the functionality of the original, healthy vessel and/or preserve any remaining vascular integrity by replacing a length of the existing vessel wall that spans the site of vessel failure. A prosthesis of this type can treat, for example, aneurysms of the abdominal aortic, iliac, or renal arteries. For instance, a prosthesis may be used to span an aneurysm which has occurred in or is associated with an iliac artery.

In many cases, such a damaged or defective portion of the vasculature may include a branch vessel. For example, the celiac, superior mesenteric, left common carotid, and renal arteries are branch vessels of the aorta, and the internal iliac artery is a branch vessel of the common iliac artery. If the branch vessel is blocked by the prosthesis, the original blood circulation is impeded, and the patient can suffer. If, for example, the celiac artery is blocked by the prosthesis, the patient can experience abdominal pain, weight loss, nausea, bloating, and loose stools associated with mesenteric ischemia. The blockage of any branch vessel is usually associated with unpleasant or even life-threatening symptoms. Therefore devices suitable for blocking the mouth of the aneurysm while maintaining vessel patency of associated branch vessels are desired.

Graft-assemblies having partial covers and devices for delivering the same have been proposed; however, new graft devices and methods of deploying the same within the vasculature of a patient are desired.

Document WO2009019664 A2 describes an implantable assembly comprising a substantially tubular expandable frame and a sheet of material covering a portion of the surface area of frame. The implantable assembly can be loaded in a non-expanded state inside a distal end of a sheath. The sheath has a slot at its distal end. A guidewire can enter the slot of the sheath to act as a reference so as to radially orient the sheath with regards to the neck of an aneurysm.

Document US2011160833 A1 describes a delivery system configured to control radial orientation of a graft to be positioned across the neck of the aneurysm with reference to an orientation guide wire. The system comprises a catheter with a distal guidewire port.

### SUMMARY

According to the present invention, there is provided a device and a system as defined in the appended claims.

For the ease of the reader, the following disclosure has been described with reference to closing the opening or mouth of a berry aneurysm; however, it will be appreciated that the devices and methods disclosed herein may be used to block and/or occlude other types of aneurysms or openings in a vessel wall, such as an opening to a branch vessel. The present disclosure provides, in certain aspects, unique devices and methods for closing a mouth of an intracranial aneurysm; however, the disclosed devices and methods can be used to close the openings to aneurysms in other locations in the vasculature of a patient. In accordance with some forms of the disclosure, such devices and methods are arranged to orient a portion of an endoluminal prosthesis with the mouth of the aneurysm so as to not block an opening of an adjacent branch vessel.

According to an embodiment of the invention, there is provided a device for deploying a stent within the vasculature of a patient, comprising a sheath having a distal end region, a proximal end region, and a sidewall extending between the distal and proximal end regions. The sidewall defines a lumen extending along a length of the sheath and a side aperture extending through the sidewall and communicating with the lumen.

The side aperture has a portion positioned proximally of the distal end region of the sheath. The sheath is arranged to retain a stent for delivery within the vasculature of the patient; and the side aperture is arranged to slidably receive an aligning member. The side aperture comprises a slot that extends along a length of the sheath. The slot tapers along a length of the slot from a distal slot end to a proximal slot end. In many embodiments, the side aperture and/or the lumen is arranged to slidably receive an aligning member comprising a wire guide. In other instances, the side aperture can be arranged to slidably receive an aligning member that is fixedly coupled to the stent graft positioned within the lumen of the sheath.

In some arrangements, a system for delivering a stent graft useful for closing a mouth of an aneurysm in a vessel of a patient comprises both an aligning member slidably coupled to the stent graft and an aligning member fixedly coupled to or integrally formed with the stent graft. For example, the stent graft may be slidably coupled to a wire guide and have an atraumatic wire loop coupled to the supporting structure of the stent graft.

The systems of the present disclosure may also include one or more trigger wires that retain portions of the stent graft to the delivery member, such as a carrier member. The trigger wire(s) are arranged for selective operation so that one or more portions of the stent graft may be deployed from the stent graft delivery member. For example, the trigger wire(s) may extend to retain one or more end portions of the supporting structure to a carrier member by extending around a portion of the supporting structure and/or capturing a portion of the supporting structure between the trigger wire(s) and a surface of the carrier member.

Further forms, objects, features, aspects, benefits, advantages, and embodiments of the present disclosure will become apparent from a detailed description and drawings provided herewith.

### BRIEF DESCRIPTION OF THE DRAWINGS

FIG. 1 illustrates a perspective view of a stent graft positioning device positioned within a vessel with an aneurysm.
FIG. 2 illustrates a top plan view of the stent graft delivery device illustrated in FIG. 1.
FIG. 3 illustrates a partial cross-sectional view of a stent graft delivery device.
FIG. 4 illustrates a side-elevational view of an exemplary stent graft with an aligning member.
FIG. 5 illustrates the exemplary stent graft of FIG. 4 positioned within a delivery sheath.
FIG. 6 illustrates another embodiment of a stent graft positioned within a delivery sheath with a wire guide extending therethrough.
FIG. 7a illustrates a top plan view of a covering material of a stent graft.
FIG. 7b illustrates a side, cross-sectional view of the embodiment of FIG. 7a.
FIG. 8a illustrates a cross-sectional view of a portion of a delivery system having stent graft preloaded within a peel-away sheath and inserted into a delivery sheath.
FIG. 8b illustrates a top view of a sheath.
FIG. 8c illustrates a cross-sectional view of a sheath.
FIG. 9a illustrates a perspective view of a supporting structure for a stent graft.
FIG. 9b illustrates a top plan view of the embodiment illustrated in FIG. 9a.
FIG. 9c illustrates the supporting structure of FIG. 9a with a covering material, so as to form a stent graft.
FIG. 9d illustrates a perspective view of an alternative embodiment of the stent graft illustrated in FIGs. 9a-9c, the stent graft further comprising an aligning member.
FIG. 10 illustrates a perspective view of another stent graft arrangement.
FIG. 11a illustrates a perspective view of another stent graft embodiment.
FIG. 11b illustrates a top plan view of the embodiment illustrated in FIG. 11a.
FIG. 12a illustrates a perspective view of a stent graft embodiment having an aligning member attached thereto.
FIG. 12b illustrates a top plan view of the embodiment illustrated in FIG. 12a.
FIG. 13 illustrates an embodiment of a stent graft comprising an aligning member.
FIG. 14 illustrates the embodiment of FIG. 13 positioned within the vasculature of a patient and blocking the mouth of an opening of an aneurysm.
FIGs. 15a, 15b, 15c, 15d, 15e and 15f illustrate a method of forming a supporting structure for an exemplary stent graft.
FIG. 16 illustrates a top plan view of the exemplary stent graft formed in FIGs. 15a-15f.
FIG. 17 illustrates a perspective view of another exemplary stent graft.
FIG. 18 illustrates a perspective view of the embodiment illustrated in FIG. 17 further comprising an aligning member.
FIG. 19 illustrates a perspective view of the stent graft embodiment illustrated in FIG. 17 with a wire guide extending through the covering material.
FIG. 20 illustrates a perspective view of the exemplary stent graft illustrated in FIG. 18 positioned on a carrier member for delivery within the vasculature of patient.

### DESCRIPTION OF THE ILLUSTRATED EMBODIMENTS

For the purpose of promoting an understanding of the principles of the disclosure, reference will now be made to the embodiments illustrated in the drawings and specific language will be used to describe the same. It will nevertheless be understood that no limitation of the scope of the disclosure is thereby intended. Any alterations and further modifications in the described embodiments, and any further applications of the principles of the disclosure as described herein are contemplated as would normally occur to one skilled in the art to which the disclosure relates.

With respect to the specification and claims, it should be noted that the singular forms "a", "an", "the", and the like include plural referents unless expressly discussed otherwise. As an illustration, references to "a device" or "the device" include one or more of such devices and equivalents thereof. It also should be noted that directional terms, such as "up", "down", "top", "bottom", and the like, are used herein solely for the convenience of the reader in order to aid in the reader's understanding of the illustrated embodiments, and it is not the intent that the use of these directional terms in any manner limit the described, illustrated, and/or claimed features to a specific direction and/or orientation.

The disclosed embodiments and variations thereof may be used to deliver at least one endoluminal prosthesis, such as a stent graft, to a location within the vasculature of a human and/or a veterinary patient. In particular, devices, systems, and methods for deploying a stent graft useful for closing a mouth of an aneurysm are disclosed. For simplicity, the following embodiments are discussed with reference to particular vessels in the body of a human patient; however, it is not intended that the present disclosure be limited to such.

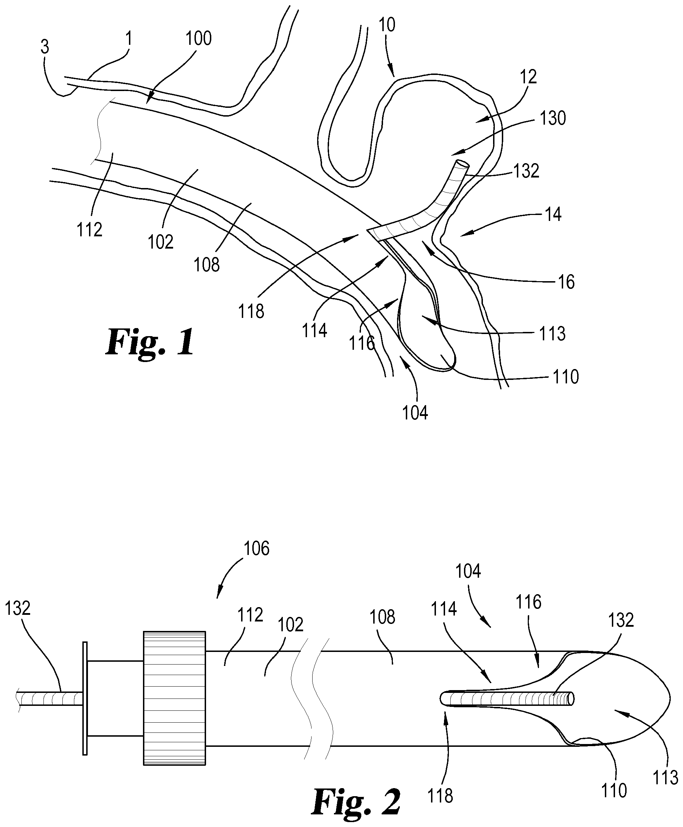

Figure 1 illustrates a stent graft delivery device 100 comprising a sheath 102 having a distal end region 104, a proximal end region 106 and a sidewall 108. Sidewall 108 defines a lumen 113 arranged to receive and retain a stent graft therein for advancement through a vessel 1 of a patient to a target location.

Device 100 may be arranged for positioning proximate an opening in a vessel wall 3, such as aneurysm 10, of a patient's vasculature. Openings such as aneurysm 10 can comprise an aneurismal sac 12, a neck 14 and a mouth 16. It should be appreciated that devices and methods of the present disclosure may be used to block the opening, such as mouth 16, of an aneurysm 10 with or without a neck 14.

Sidewall 108 of sheath 102 comprises an inner surface 110 and an outer surface 112. Inner surface 110 defines lumen 113 that extends along a length of sheath 102. Preferably, in some instances, sidewall 108 defines one or more side apertures, such as a slot 114, that communicate with lumen 113.

In some embodiments, slot 114 has a distal slot end 116 and a proximal slot end 118 and may taper along a length of slot 114, such as tapering from a wide configuration to a narrow configuration from distal slot end 116 towards proximal slot end 118.

In many instances, the side aperture defined by sidewall 108, such as slot 114, is arranged to receive an aligning member 130, such as a wire guide 132, arranged to align the sheath 102 with the aneurysm 10. For example, wire guide 132 may extend through the vasculature of the patient towards aneurysm 10 and the distal tip of wire guide 132 positioned within aneurismal sac 12. Sheath 102 may then be advanced over wire guide 132, with wire guide 132 slidably extending through lumen 113. As sheath 102 is advanced over wire guide 132, the slot 114 of sidewall 108 receives wire guide 132. As sheath 102 is advanced so as to positioned wire guide 132 near proximal slot end 118, the angle between wire guide 132 and the longitudinal axis of the distal end region 104 of sheath 102 increases, and sheath 102 rotates into an orientation in which slot 114 opens towards the mouth 16 of aneurysm 10. Advantageously, in some embodiments, sheath 102 will automatically rotate to align a side aperture, such as slot 114, with mouth 16 of aneurysm 10 as sheath 102 is advanced over wire guide 132.

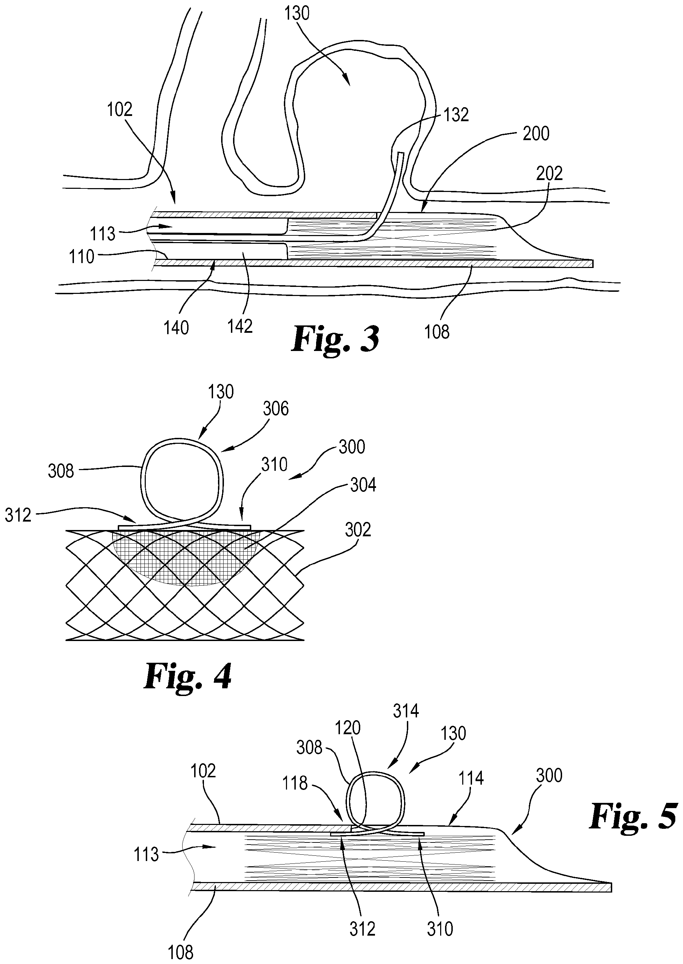

Moving on to figure 3, device 100 can comprise a pushing member 140 arranged to push a stent graft 200 comprising at least a supporting structure 202 from within the lumen 113 defined by inner surface 110 of sidewall 108. Pushing member 140 comprises pushing portions 142 arranged to contact stent graft 200 and push stent graft 200 through and/or from lumen 113. Pushing member 140 may comprise a catheter that is slidably receivable within lumen 113 and advanced over wire guide 132. Alternatively, pushing member 140 may comprise pushing portions that are coupled to wire guide 132. The pushing member is arranged so that stent graft 200 may be pushed out of lumen 113 so as to expand from an initial configuration to a deployed, expanded configuration within the vasculature of the patient. The pushing member can also be arranged for pushing a stent graft through lumen 113 along the length of sheath 102.

Figures 4 and 5 illustrate another embodiment of a stent graft 300 comprising a supporting structure 302 and a covering material 304 extending across a surface of supporting structure 302. Stent graft 300 can also comprise an aligning member 130, such as loop 306. Loop 306 can be formed by a wire 308 having a distal portion 310, a proximal portion 312 and a central portion 314 extending between the distal portion 310 and proximal portion 312. Distal portion 310 and proximal portion 312 of wire 308 are fixedly coupled to supporting structure 302 of the stent graft 300, and central portion 314 forms an atraumatic loop 306 arranged for positioning within a portion of an aneurysm 10.

When stent graft 300 is positioned within lumen 113 of a sheath 102, aligning member 130, such as atraumatic loop 306, can extend from a side aperture such as slot 114 of sheath 102. Advantageously, aligning member 130 may be arranged for positioning within mouth 16 of aneurysm 10 so that a portion of stent graft 300 aligns with aneurysm 10 prior to deployment of stent graft 300 from within lumen 113 of sheath 102. For example, in embodiments in which atraumatic loop 306 is positioned over covering material 304, atraumatic loop will extend within mouth 16 of aneurysm 10 and align covering material 304 with mouth 16 so that when stent graft 300 is deployed, covering material 304 extends across and seals mouth 16.

As can be seen in figure 5, some arrangements are free of a pushing member 140. For example, sidewall 108 may have an edge 120 positioned at the proximal slot end 118. Edge 120 may be arranged to contact portions of an aligning member 130 such as atraumatic loop 306, so as to prevent stent graft 300 from migrating proximally within lumen 113 of sheath 102 during advancement of device 100 through the vasculature of a patient. In some instances, portions of sidewall 108 such as edge 120, wedge between the proximal portion 312 and central portion 314 of wire 308 so as to keep atraumatic loop 306 from collapsing and/or moving in a proximal direction. When stent graft 300 is in the desired position within the vasculature of the patient (i.e., when loop 306 is within the target aneurysm), stent graft 300 may be deployed by withdrawing sheath 102 in a proximal direction. Alternatively or additionally, a trigger wire and/or splittable sheath may be used to secure stent graft 300 to the distal end region of sheath 102 and removed or disconnected from stent graft 300 prior to or during deployment of stent graft 300.

Advantageously, aligning member 130, such as loop 306, can be arranged to extend into the mouth 16 of the aneurysm 10 when in alignment. For example, when loop 306 aligns with the mouth 16 of the aneurysm 10, loop 306 can extend, such as into the configuration illustrated in figure 5, into the aneurysm 10 and provide a tactile response (e.g., a greater resistance to movement in one or more directions) to indicate to the medical professional that loop 306 is aligned with an opening in the vessel wall. Alternatively or additionally, loop 306 may be visualized, such as by X-ray, so that a medical professional may see that loop 306 has extended into the aneurysm and is therefore aligning with an opening in the wall of the patient's vessel.

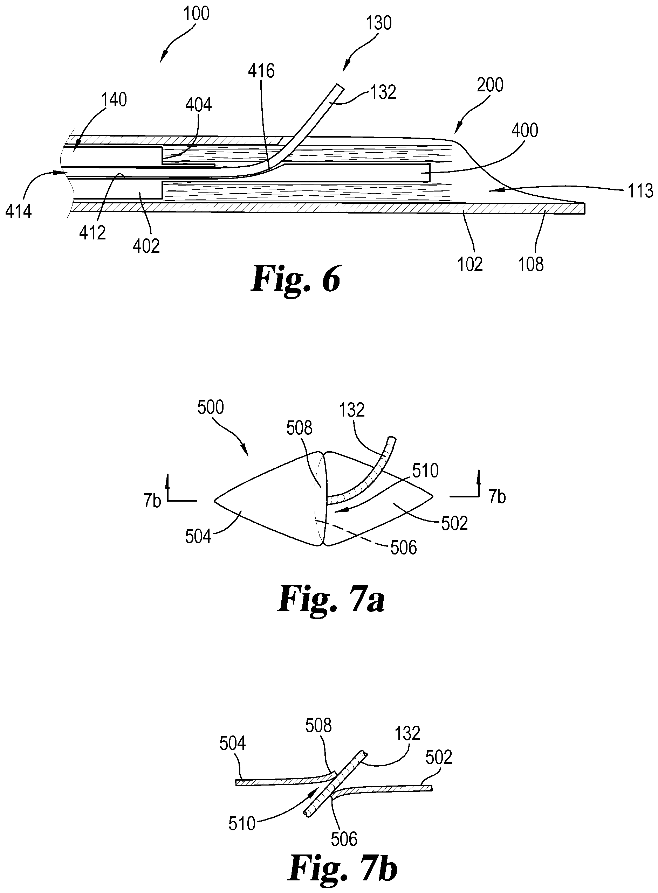

Figure 6 illustrates another embodiment of a device 100 having a stent graft 200 positioned within a lumen 113 of a sheath 102. As illustrated, the aligning member 130 can comprise a wire guide 132 that extends through lumen 113 and a side portion of stent graft 200. In some instances, device 100 further comprises a wire guide diverting member 400 positioned within lumen 113 of sheath 102. Wire guide diverting member 400 comprising a pusher 402 having a pushing surface 404 and an inner surface 412 that defines a lumen 414 for receiving wire guide 132. Wire guide diverting member 400 comprises an arcuate surface 416 arranged to divert portions of wire guide 132 through a side of stent graft 200. As discussed above, sheath 102 can be advanced over wire guide 132 until the side aperture is positioned adjacent the aneurysm, and stent graft 200 can be deployed from lumen 414 by pushing stent graft 200 with pusher 402, withdrawing sheath 102 from around stent graft 200, or a combination of pushing stent graft 200 with pusher 402 and withdrawing sheath 102.

Figures 7a and 7b illustrate one arrangement of covering material 500 comprising a first portion 502 and second portion 504 overlapping along a seam so as to form an opening arranged to receive an aligning member 130, such as wire guide 132. For example, first portion 502 may comprise a first overlapping edge portion 506 that overlaps a second overlapping edge portion 508 of second portion 504. First overlapping edge portion 506 and second overlapping edge portion 508 cooperate to form a collapsible opening 510 arranged to receive wire guide 132.

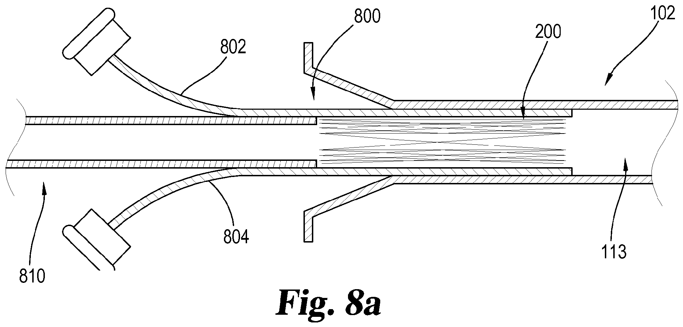

Figure 8a illustrates a portion of delivery system that is capable of delivering a stent graft, such as one of the exemplary stent grafts or delivery systems illustrated herein. As illustrated, stent graft 200 may be positioned within a peel-away sheath 800 that comprises a first portion 802 and a second portion 804. The first and second portions 802, 804 are separable (e.g., splittable) from one another so as to expose the stent graft. Preferably, peel-away sheath 800 retains stent graft 200 at the end of a pusher 810, and peel-away sheath 800 is sized and configured for insertion into the proximal opening of lumen 113 of sheath 102. After insertion of peel-away sheath 800, stent graft 200, and the distal end of pusher 810 into lumen 113, the first and second portions 802, 804 of peel-away sheath 800 may be separated from one another and removed from lumen 113. Pusher 810 may then be used to push stent graft 200 through lumen 113 and to the target location within the body of the patient. Additionally, stent graft 200 could be advanced over a wire guide.

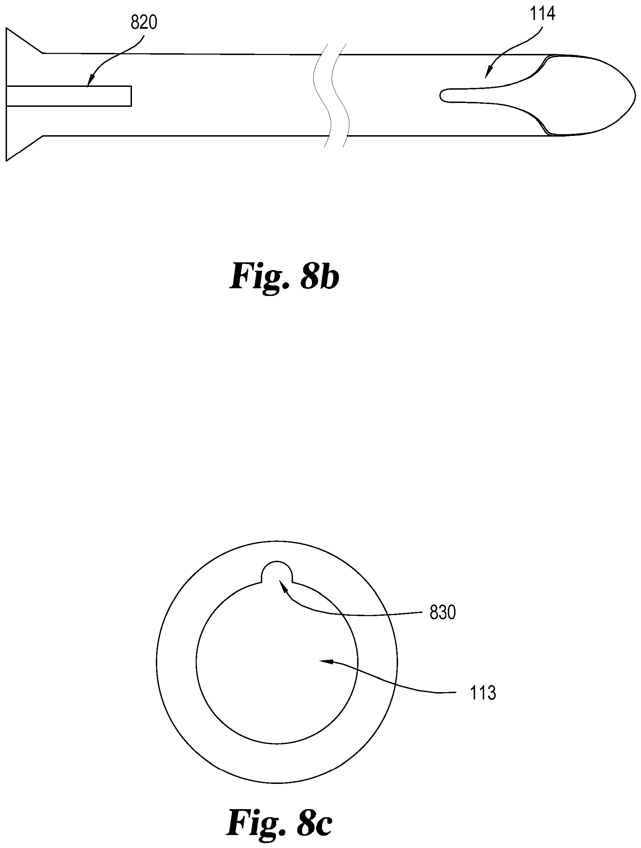

Figure 8b illustrates a sheath having an external marker 820 positioned along the proximal end of the sheath, such as on a handle of the sheath. External marker 820 is aligned with a side aperture, such as slot 114, that is positioned at the distal end region of the sheath. External marker therefore allows a medical professional to know the orientation of the side aperture when the distal end region of the sheath is positioned within the body of a patient.

Figure 8c illustrates a cross-sectional view of a sheath having a groove 830 arranged to receive an aligning member. Groove 830 preferably aligns with and/or terminates at a side aperture (e.g., slot 114) in the distal end region of the sheath. Additionally, groove 830 can align with an external marker, such as external marker 820, positioned on the handle and/or proximal end region of the sheath. As a stent graft is advanced through lumen 113 of the sheath, the aligning member that is coupled to (e.g., slidably or fixedly coupled) or integrally formed with the stent graft, tracks groove 830 and prevents rotation of the stent graft within lumen 113. This arrangement can therefore maintain alignment of the stent graft with the sheath during advancement of the stent graft through the lumen of the sheath.

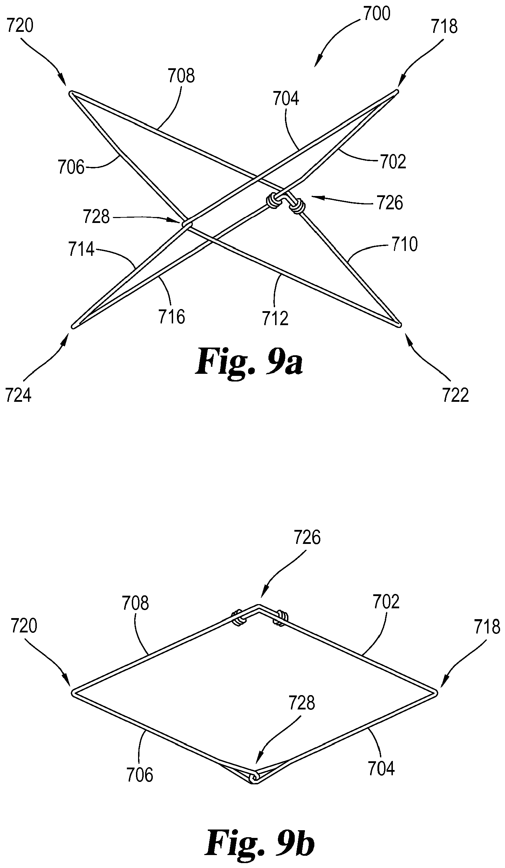

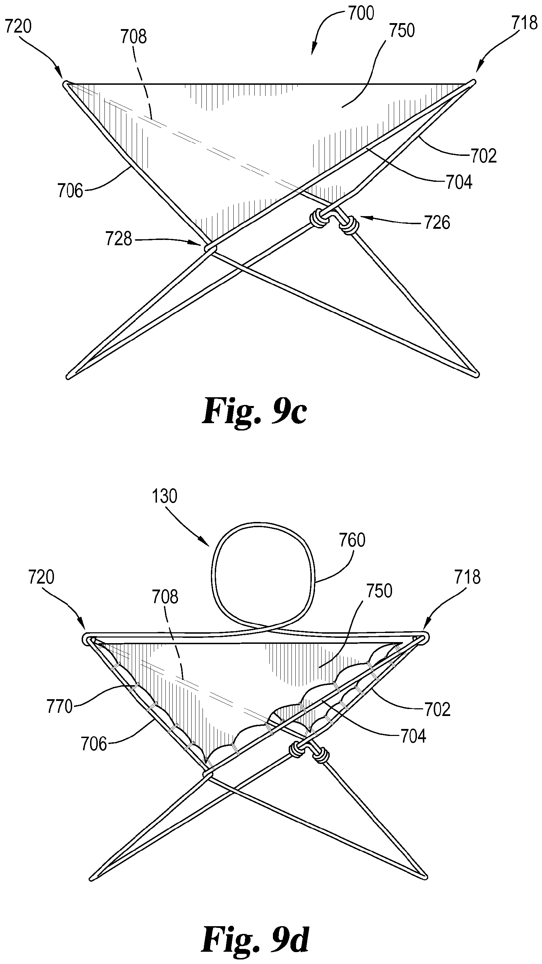

Figures 9a, 9b and 9c illustrate a frame or supporting structure 700 of a stent graft suitable for closing an opening of an aneurysm. In some instances, supporting structure 700 comprises an elongate member such as a single, continuous wire portion that is bent into an expandable configuration. For example, an elongate member may be bent so as to form a plurality of struts, such as struts 702, 704, 706, 708, 710, 712, 714 and 716, with the struts connected by bends 718, 720, 722 and 724. Adjacent bends can be connected at bend junctions 726, 728. Preferably, supporting structure 700 is arranged to self-expand when released from within a lumen 113 of a sheath 102.

Figure 9c illustrates the supporting structure 700 having a covering material 750 extending across a portion thereof. For example, covering material 750 may extend along struts 702, 704, 706 and 708 and between bends 718, 720 so that the covering material forms a covering across a top portion of supporting structure 700.

Figure 9d illustrates a supporting structure 700 having an aligning member 130 integrally formed or attached thereto. For example, aligning member 130 may comprise a wire 760 extending from bend 718 to bend 720 and forming atraumatic loop across a central portion thereof. Figure 9d also illustrates that covering material 750 may be attached to portions of supporting structure 700, such as struts, by one or more sutures 770.

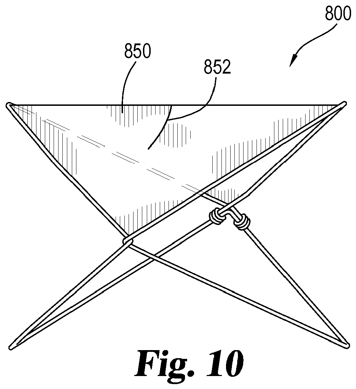

Figure 10 illustrates a stent graft arrangement in which the supporting structure is constructed of a single, continuous wire and is arranged so that a path extending from the area within the stent graft through the patch is free of struts. For example, the stent graft may comprise a supporting structure 800 having a covering material 850 extending across a portion thereof. Covering material 850 may define a closable opening 852 (e.g., a slit) arranged to receive a wire guide. For example, supporting structure 800 and covering material 850 may be arranged for sliding advancement over a portion of a wire guide positioned within slit 852. In some exemplary methods of deploying a stent graft, a supporting structure and covering material may be advanced over a wire guide that has a portion positioned within an aneurysm or vessel target for closure. The closable opening in the covering material may be similar or the same as that illustrated and described with respect to figures 7a and 7b and elsewhere in this application.

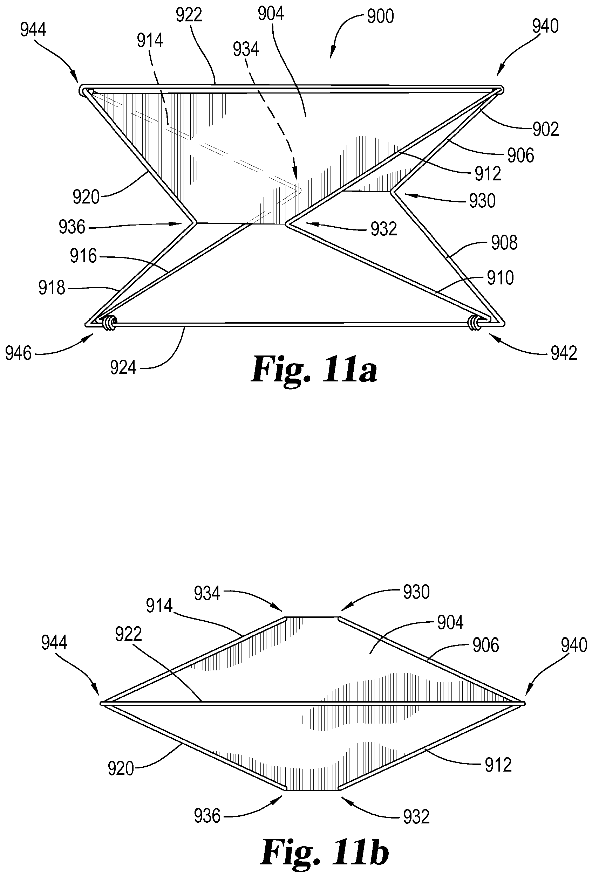

Figures 11a and 11b illustrate another embodiment of a stent graft 900 comprising supporting structure 902 and a covering material 904. Stent graft 900 has a greater resistance to compression along a longitudinal axis than the embodiment illustrated in figures 9a-9c and maintains the same device length before and after expansion. As illustrated in figures 11a and 11b, supporting structure 902 of stent graft 900 comprises struts 906, 908, 910 and 912 forming a z-stent arrangement at one end of the stent graft 900 and struts 914, 916, 918 and 920 forming a second z-stent arrangement at an opposing end of the stent graft 900. As described with regards to the supporting structure embodiments described above, portions of supporting structure 902 may be formed from one or more elongate members. For example, a single elongate member may be bent at bends 930 and/or 932. Similarly, a single elongate member may form the z-stent arrangement at the opposing end, being bent at bends 934, 936. The two z-stents of supporting structure 902 may be coupled to one another by a top strut 922 and a bottom strut 924, coupling first and second z-stents at junctions 940 and 944 along a top portion, and junctions 942, 946 along a bottom portion of the stent graft 900. Top and bottom struts 922 and 924 prevent the stent graft from changing length before and after deployment.

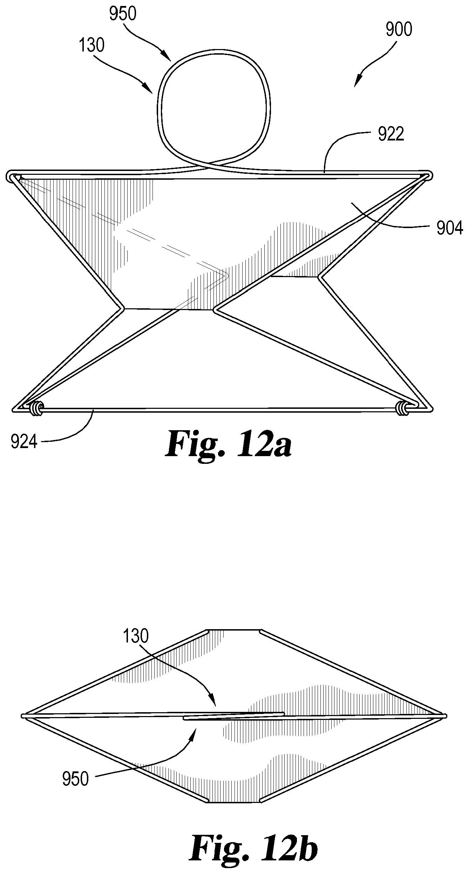

Figures 12a and 12b illustrate a variation of the stent graft 900 illustrated in figures 11a and 11b. In figures 12a and 12b, top strut 922 of stent graft 900 forms an atraumatic loop 950 arranged to function as the aligning member 130 for stent graft 900. Like bottom strut 924 in the above embodiment, bottom strut 924 prevents the stent graft from changing length before and after deployment. Additionally, covering material 904 may define a slit, such as one of those illustrated and described in figures 7a, 7b, and 10. The slit can be arranged to receive a wire guide and allow the slidable movement of the covering material over the wire guide so that the covering material may be placed over the opening of a target aneurysm in which a distal portion of the wire guide is positioned.

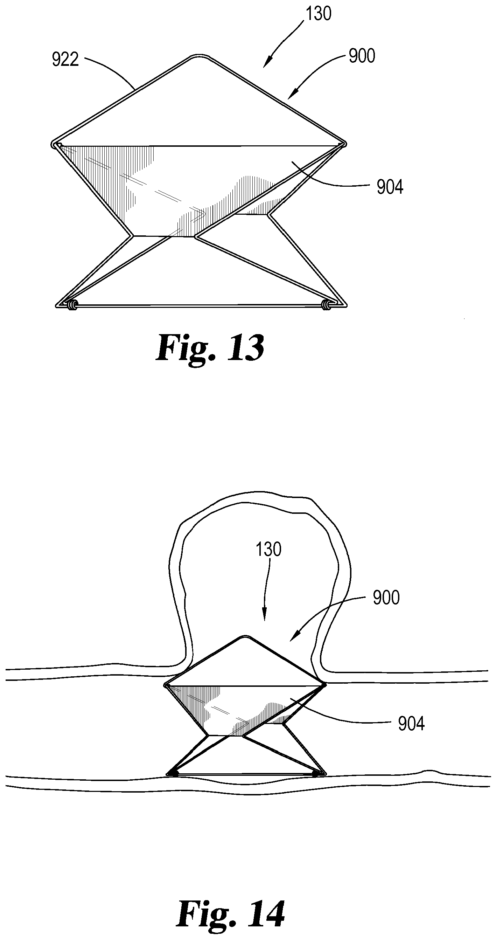

Figures 13 and 14 illustrate another embodiment of stent graft 900 comprising an aligning member. In this embodiment, aligning member 130 comprises top strut 922 bent into a triangular configuration. Figure 14 illustrates a stent-graft, such as the embodiment of figure 13, positioned within a vessel of a patient, with the aligning member 130 extending into the aneurysm and aligning the covering material 904 with the mouth of the aneurysm so as to at least partially occlude and/or close the mouth of the aneurysm. Advantageously, the covering material of the stent graft can sufficiently resist blood flow into the aneurysm so as to decrease the pressure within the aneurysm and/or prevent blood flow into the aneurysm.

As will be appreciated by those of ordinary skill in the art, an aligning member may be positioned radially outward and/or radially inward of the covering material of the stent graft. For example, the end portions of the top strut 922 may be positioned radially inward (e.g., underneath) of the covering material with the central portion of the top strut 922, such as the atraumatic loop 950, extending through an opening in the covering material so as to have a portion that is positioned radially outward (e.g., above) of the outer surface of the covering material. In some embodiments, the aligning member may be positioned substantially underneath or substantially above the covering material.

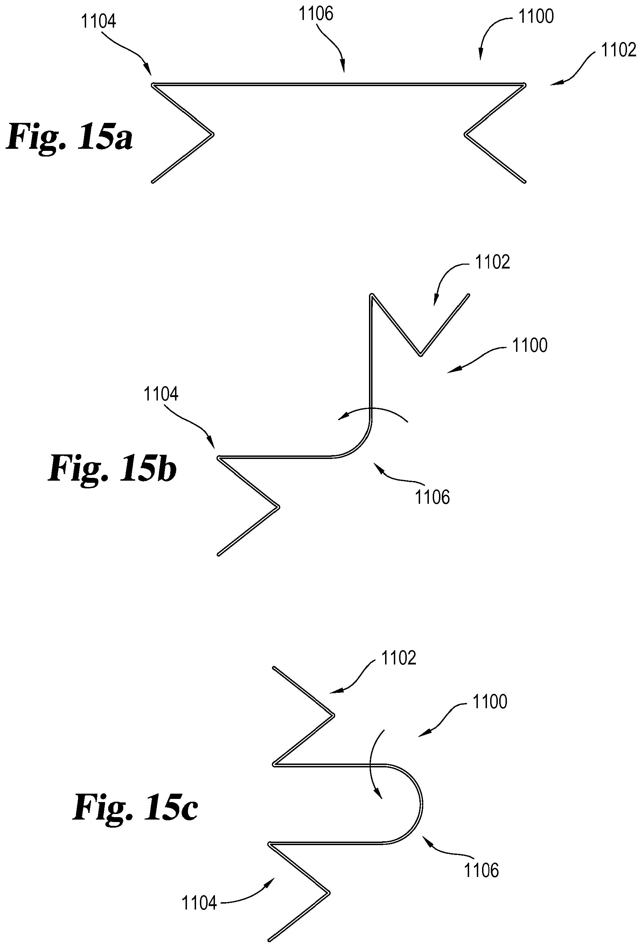

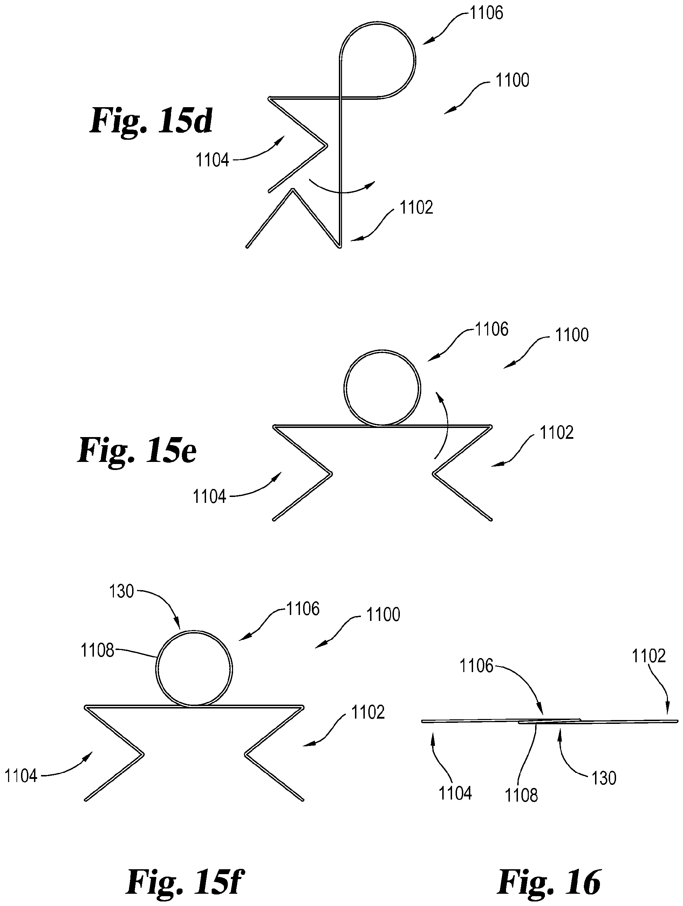

Figures 15a-15f illustrate one method of forming a frame for a stent graft suitable for closing the mouth of an aneurysm. In figure 15a, frame 1100 comprises a distal end region 1102, a proximal end region 1104 and a central region 1106 extending between distal end region 1102 and proximal end region 1104. To form an atraumatic loop 1108 suitable to function as an aligning member 130, distal end region 1102 of frame 1100 is bent upwards towards proximal end region 1104, as illustrated in figure 15b. As distal end region 1102 is bent towards proximal end region 1104, central region 1106 forms a loop arrangement, shown in figures 15c-15d, which is closed upon completion of a full rotation by distal end region 1102 around central region 1106. figure 16 illustrates a top plan view of frame 1100 formed using the method illustrated in figures 15a-15f.

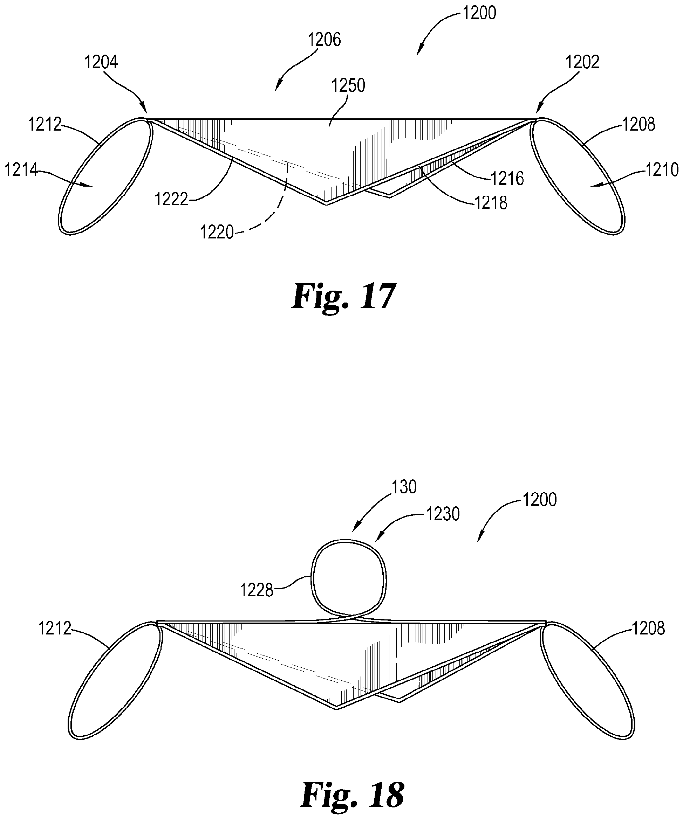

Figures 17-20 illustrate additional embodiments of a stent graft 1200 arranged for closing the mouth of an aneurysm. Stent graft 1200 can comprise a distal end region 1202, a proximal end region 1204, and a central region 1206 extending between distal end region 1202 and proximal end region 1204. Positioned at distal end region 1202 is wire member 1208 forming a loop configuration that defines an opening 1210 arranged to permit unobstructed flow of fluid through the lumen of a vessel when stent graft 1200 is deployed. Alternatively or additionally, opening 1210 defined by the loop configuration can be arranged to receive a carrier member for delivery of stent graft 1200. Similarly, positioned at proximal end region 1204 is a wire member 1212 forming a loop configuration defining an opening 1214 similar to that of wire member 1208 and opening 1210. Central region 1206 of stent graft 1200 comprises struts 1216, 1218, 1220 and 122 arranged to extend a covering material 1250 across central region 1206 of stent graft 1200.

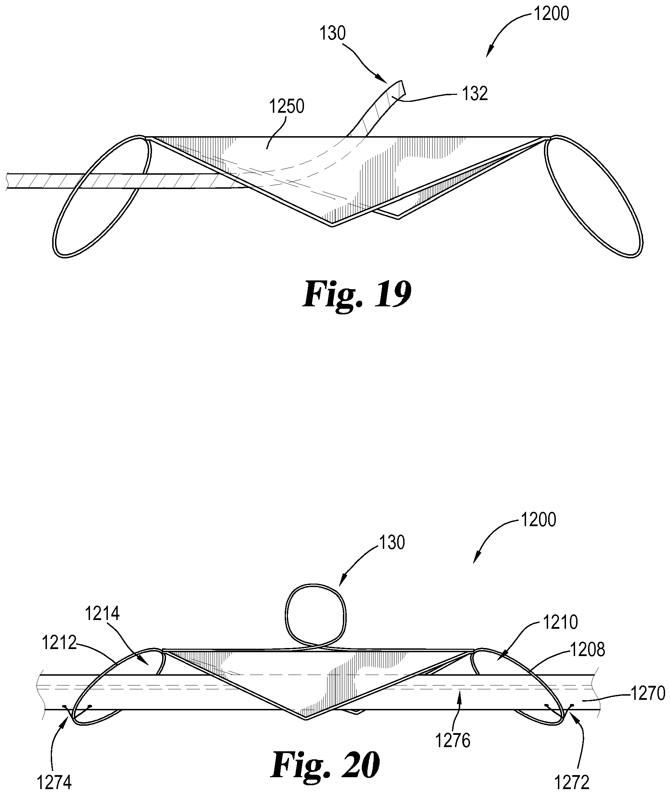

In some instances, stent graft 1200 comprises a wire member 1228 extending from distal end region 1202 to proximal end region 1204 and forming an atraumatic loop 1230 in central region 1206. In some embodiments, it is preferred that the atraumatic loop 1230 formed by wire member 1228 is positioned over covering material 1250, so that the atraumatic loop 1230 of wire member 1228 can serve as an aligning member 130 to align covering material 1250 with the mouth of an aneurysm. Alternatively or additionally, stent graft 1200 may use a wire guide 132 as an aligning member 130, the wire guide 132 extending through an opening defined by covering material 1250 and into an aneurysm so as to align covering material 1250 of stent graft 1200 with the mouth of the aneurysm (illustrated in figure 19).

Figure 20 illustrates an exemplary arrangement in which a stent graft, such as stent graft 1200, is positioned on a carrier member 1270 arranged for delivering stent graft 1200 to a target location within the body of a patient. For example, carrier member 1270 may comprise an elongate body such as a catheter arranged for positioning through openings 1210 and 1214 defined by wire members 1208 and 1212 of stent graft 1200. In some instances, carrier member 1270 includes a distal trigger wire 1272 coupled to wire member 1208 near the distal end region 1202 of the stent graft 1200. Similarly, carrier member 1270 may include a proximal trigger wire 1274 coupled to wire member 1212 at proximal end region 1204 of stent graft 1200 so that stent graft 1200 may be stretched along a longitudinal axis by trigger wires 1272 and 1274 to decrease the profile of stent graft 1200. As will be appreciated by those of ordinary skill in the art, carrier member 1270 may comprise one or more trigger wire lumens 1276 arranged to receive one or more of the trigger wires 1272, 1274 for operation of one or more of the trigger wires 1272, 1274 at the proximal end of the carrier member 1270 located outside the body of the patient of carrier member 1270.

While at least one embodiment has been illustrated and described in detail in the drawings and foregoing description, the same is to be considered as illustrative and not restrictive in character, it being understood that the preferred embodiment has been shown and described and that all changes, equivalents, and modifications that come within the invention as defined by the following claims are desired to be protected. It will be evident from the specification that aspects or features discussed in one context or embodiment will be applicable in other contexts or embodiments.

## Claims

1. A device (100) for deploying a stent (200) within the vasculature of a patient, comprising:
a sheath (102) having a distal end region (104), a proximal end region (106), and a sidewall (108) extending between said distal and proximal end regions;
said sidewall (108) defining a lumen (113) extending along a length of said sheath (102) and a side aperture extending through said sidewall (108) and communicating with said lumen (113);
said side aperture having a portion positioned proximally of said distal end region (104) of said sheath (102);
wherein said sheath (102) is arranged to retain a stent for delivery within the vasculature of the patient;
wherein said side aperture is arranged to slidably receive an aligning member (130); and
wherein said side aperture comprises a slot (114) extending along a length of said sheath (102);
**characterized in that** said slot (114) comprises a distal slot end (116) and a proximal slot end (118) and tapers along a length of said slot (114) from said distal slot end (116) to said proximal slot end (118).

2. The device of claim 1, wherein:
said portion of said sheath (102) comprising said side aperture is rotatably coupled to said proximal end of said sheath (102).

3. A system comprising the device (100) of claim 1, a stent graft (200) slidably positioned within said lumen (113), and an aligning member (130);
said stent graft (200) having a supporting structure (202) expandable between a first configuration arranged for delivery of said stent graft (200) with the delivery member (100) and a second configuration arranged for deployment in a vessel (1);
said stent graft (200) having a covering material (500) extending along a portion of said stent graft (200); and
said aligning member (130) coupled to said stent graft (200) and extending from an area of said stent graft (200) covered by said covering material (500).

4. The system of claim 3, wherein:
said aligning member (130) is arranged to extend from said side aperture beyond said sidewall (108) of said sheath (102).

5. The system of claim 3 or claim 4, wherein:
said covering material (500) extends across only a portion of said supporting structure (202).

6. The system of any one of claims 3-5, wherein:
said covering material (500) of said stent graft (200) is aligned with said side aperture of said sheath (102) when said stent graft (200) is positioned within said lumen (113).

7. The system of any one of claims 3-6, wherein:
said aligning member (130) and said stent graft (200) are slidably coupled so that said stent graft (200) can slide along a length of said aligning member (130).

8. The system of any one of claims 3-7, wherein:
said aligning member (130) and said stent graft (200) are coupled so that said stent graft (200) follows said aligning member (130) when said stent graft (200) expands between said first and second configurations.

9. The system of any one of claims 3-8, wherein:
said stent graft (200) has the same length in said first configuration and said second configuration.

10. The system of any one of claims 3-9, wherein:
said aligning member (130) comprises a wire guide (132) slidably extending through a portion of said stent graft (200).

11. The system of claim 10, wherein:
said aligning member (130) extends through an opening in said covering material (500).

12. The system of claim 11, wherein:
said opening comprises separable, overlapping portions (502, 504) of said covering material (500).

13. The system of any one of claims 3-12, wherein:
said aligning member (130) is fixedly coupled to said stent graft (200) and is arranged to extend away from said stent graft (200).

14. The system of claim 13, wherein:
said aligning member (130) comprises a wire (132).

15. The system of any one of claims 9-14, wherein:
said aligning member (130) is arranged to prevent a misaligning movement of said stent graft (200) within the vessel (1) of the patient while said supporting structure (202) expands into said second configuration.

## Patentansprüche

1. Eine Vorrichtung (100) zum Ablegen eines Stents (200) im Gefäßsystem eines Patienten, die Folgendes beinhaltet:
eine Schleuse (102) mit einer distalen Endregion (104), einer proximalen Endregion (106) und einer Seitenwand (108), die sich zwischen der distalen und proximalen Endregion erstreckt;
wobei die Seitenwand (108) ein Lumen (113), das sich entlang einer Länge der Schleuse (102) erstreckt, und eine Seitenöffnung, die sich durch die Seitenwand (108) erstreckt und in Verbindung mit dem Lumen (113) steht, definiert;
wobei die Seitenöffnung einen Anteil aufweist, der proximal zu der distalen Endregion (104) der Schleuse (102) liegt;
wobei die Schleuse (102) zum Halten eines Stents, der in das Gefäßsystem des Patienten zugeführt werden soll, eingerichtet ist;
wobei die Seitenöffnung zum gleitfähigen Aufnehmen eines Ausrichtungselements (130) eingerichtet ist; und
wobei die Seitenöffnung einen Schlitz (114) beinhaltet, der sich entlang einer Länge der Schleuse (102) erstreckt;
**dadurch gekennzeichnet, dass** der Schlitz (114) ein distales Schlitzende (116) und ein proximales Schlitzende (118) beinhaltet und sich entlang einer Länge des Schlitzes (114) von dem distalen Schlitzende (116) zu dem proximalen Schlitzende (118) verjüngt.

2. Vorrichtung nach Anspruch 1, wobei:
der Anteil der Schleuse (102), die die Seitenöffnung beinhaltet, drehbar mit dem proximalen Ende der Schleuse (102) gekoppelt ist.

3. Ein System, das die Vorrichtung (100) nach Anspruch 1, eine gleitfähig im Innern des Lumens (113) positionierte Stentprothese (200) und ein Ausrichtungselement (130) beinhaltet;
wobei die Stentprothese (200) eine Stützstruktur (202) aufweist, die zwischen einer ersten Konfiguration, die zum Zuführen der Stentprothese (200) mit dem Zuführungselement (100) eingerichtet ist, und einer zweiten Konfiguration, die zum Ablegen in einem Gefäß (1) eingerichtet ist, expandierbar ist;
wobei die Stentprothese (200) ein Abdeckungsmaterial (500) aufweist, das sich entlang eines Anteils der Stentprothese (200) erstreckt;
und wobei das Ausrichtungselement (130) mit der Stentprothese (200) gekoppelt ist und sich aus einem durch das Abdeckungsmaterial (500) abgedeckten Bereich der Stentprothese (200) erstreckt.

4. System nach Anspruch 3, wobei:
das Ausrichtungselement (130) dazu eingerichtet ist, sich aus der Seitenöffnung über die Seitenwand (108) der Schleuse (102) hinaus zu erstrecken.

5. System nach Anspruch 3 oder Anspruch 4, wobei:
sich das Abdeckungsmaterial (500) nur über einen Anteil der Stützstruktur (202) erstreckt.

6. System nach einem der Ansprüche 3-5, wobei:
das Abdeckungsmaterial (500) der Stentprothese (200) mit der Seitenöffnung der Schleuse (102) ausgerichtet ist, wenn die Stentprothese (200) innerhalb des Lumens (113) positioniert ist.

7. System nach einem der Ansprüche 3-6, wobei:
das Ausrichtungselement (130) und die Stentprothese (200) gleitfähig gekoppelt sind, sodass die Stentprothese (200) an einer Länge des Ausrichtungselements (130) entlanggleiten kann.

8. System nach einem der Ansprüche 3-7, wobei:
das Ausrichtungselement (130) und die Stentprothese (200) gekoppelt sind, sodass die Stentprothese (200) dem Ausrichtungselement (130) folgt, wenn die Stentprothese (200) zwischen der ersten und zweiten Konfiguration expandiert.

9. System nach einem der Ansprüche 3-8, wobei:
die Stentprothese (200) in der ersten Konfiguration und der zweiten Konfiguration die gleiche Länge aufweist.

10. System nach einem der Ansprüche 3-9, wobei:
das Ausrichtungselement (130) eine Drahtführung (132) beinhaltet, die sich gleitfähig durch einen Anteil der Stentprothese (200) erstreckt.

11. System nach Anspruch 10, wobei:
sich das Ausrichtungselement (130) durch ein Loch in dem Abdeckungsmaterial (500) erstreckt.

12. System nach Anspruch 11, wobei:
das Loch trennbare, überlappende Anteile (502, 504) des Abdeckungsmaterials (500) beinhaltet.

13. System nach einem der Ansprüche 3-12, wobei:
das Ausrichtungselement (130) fest mit der Stentprothese (200) gekoppelt ist und dazu eingerichtet ist, sich von der Stentprothese (200) weg zu erstrecken.

14. System nach Anspruch 13, wobei:
das Ausrichtungselement (130) einen Draht (132) beinhaltet.

15. System nach einem der Ansprüche 9-14, wobei:
das Ausrichtungselement (130) dazu eingerichtet ist, während des Expandierens der Stützstruktur (202) in die zweite Konfiguration eine Fehlausrichtungsbewegung der Stentprothese (200) innerhalb des Gefäßes (1) des Patienten zu verhindern.

## Revendications

1. Dispositif (100) destiné à déployer une endoprothèse (200) au sein du système vasculaire d'un patient, comprenant :
une gaine (102) ayant une région d'extrémité distale (104), une région d'extrémité proximale (106), et une paroi latérale (108) s'étendant entre lesdites régions d'extrémités distale et proximale ;
ladite paroi latérale (108) définissant une lumière (113) s'étendant le long d'une longueur de ladite gaine (102) et une ouverture latérale s'étendant à travers ladite paroi latérale (108) et communiquant avec ladite lumière (113) ;
ladite ouverture latérale ayant une partie positionnée de manière proximale par rapport à ladite région d'extrémité distale (104) de ladite gaine (102) ;
dans lequel ladite gaine (102) est agencée pour retenir une endoprothèse destinée à être délivrée au sein du système vasculaire du patient ;
dans lequel ladite ouverture latérale est agencée pour recevoir de manière coulissante un élément d'alignement (130) ; et
dans lequel ladite ouverture latérale comprend une fente (114) s'étendant le long d'une longueur de ladite gaine (102) ;
**caractérisé en ce que** ladite fente (114) comprend une extrémité de fente distale (116) et une extrémité de fente proximale (118) et s'effile le long d'une longueur de ladite fente (114) de ladite extrémité de fente distale (116) à ladite extrémité de fente proximale (118).

2. Dispositif selon la revendication 1, dans lequel :
ladite partie de ladite gaine (102) comprenant ladite ouverture latérale est couplée de manière rotative à ladite extrémité proximale de ladite gaine (102).

3. Système comprenant le dispositif (100) de la revendication 1, une endoprothèse-greffon (200) positionnée de manière coulissante au sein de ladite lumière (113), et un élément d'alignement (130) ;
ladite endoprothèse-greffon (200) ayant une structure de support (202) capable de développement entre une première configuration agencée pour la délivrance de ladite endoprothèse-greffon (200) avec l'élément de délivrance (100) et une deuxième configuration agencée pour le déploiement dans un vaisseau (1) ;
ladite endoprothèse-greffon (200) ayant un matériau de couverture (500) s'étendant le long d'une partie de ladite endoprothèse-greffon (200) ;
et ledit élément d'alignement (130) couplé à ladite endoprothèse-greffon (200) et s'étendant depuis une surface de ladite endoprothèse-greffon (200) recouverte par ledit matériau de couverture (500).

4. Système selon la revendication 3, dans lequel :
ledit élément d'alignement (130) est agencé pour s'étendre depuis ladite ouverture latérale au-delà de ladite paroi latérale (108) de ladite gaine (102).

5. Système selon la revendication 3 ou la revendication 4, dans lequel :
ledit matériau de couverture (500) s'étend sur une partie uniquement de ladite structure de support (202).

6. Système selon l'une quelconque des revendications 3 à 5, dans lequel :
ledit matériau de couverture (500) de ladite endoprothèse-greffon (200) est aligné avec ladite ouverture latérale de ladite gaine (102) quand ladite endoprothèse-greffon (200) est positionnée au sein de ladite lumière (113) .

7. Système selon l'une quelconque des revendications 3 à 6, dans lequel :
ledit élément d'alignement (130) et ladite endoprothèse-greffon (200) sont couplés de manière coulissante de sorte que ladite endoprothèse-greffon (200) peut coulisser le long d'une longueur dudit élément d'alignement (130).

8. Système selon l'une quelconque des revendications 3 à 7, dans lequel :
ledit élément d'alignement (130) et ladite endoprothèse-greffon (200) sont couplés de sorte que ladite endoprothèse-greffon (200) suit ledit élément d'alignement (130) quand ladite endoprothèse-greffon (200) se développe entre lesdites première et deuxième configurations.

9. Système selon l'une quelconque des revendications 3 à 8, dans lequel :
ladite endoprothèse-greffon (200) présente la même longueur dans ladite première configuration et ladite deuxième configuration.

10. Système selon l'une quelconque des revendications 3 à 9, dans lequel :
ledit élément d'alignement (130) comprend un guide-fil (132) s'étendant de manière coulissante à travers une partie de ladite endoprothèse-greffon (200).

11. Système selon la revendication 10, dans lequel :
ledit élément d'alignement (130) s'étend à travers une ouverture dans ledit matériau de couverture (500).

12. Système selon la revendication 11, dans lequel :
ladite ouverture comprend des parties se chevauchant, séparables (502, 504) dudit matériau de couverture (500).

13. Système selon l'une quelconque des revendications 3 à 12, dans lequel :
ledit élément d'alignement (130) est couplé fixement à ladite endoprothèse-greffon (200) et est agencé pour s'étendre à l'écart de ladite endoprothèse-greffon (200).

14. Système selon la revendication 13, dans lequel :
ledit élément d'alignement (130) comprend un fil (132).

15. Système selon l'une quelconque des revendications 9 à 14, dans lequel :
ledit élément d'alignement (130) est agencé pour prévenir un mouvement de déport d'alignement de ladite endoprothèse-greffon (200) au sein du vaisseau (1) du patient pendant que ladite structure de support (202) se développe en ladite deuxième configuration.
